# FASCICULE DE BREVET EUROPEEN

(11) **EP 1 582 198 B1**
(45) Date de publication et mention de la délivrance du brevet: **26.05.2010**
(21) Numéro de dépôt: 05300248.1
(22) Date de dépôt: 01.04.2005
(51) Int. Cl.: A61K 8/896, A61K 8/891, A61Q 5/06, A45D 7/06

(54) **Procédé de traitement des fibres capillaires à base de compositions siliconées**
Verfahren zur Behandlung von Haaren unter Verwendung von Siliconzusammensetzungen
Method for treating hair with silicone compositions

(30) Priorité: 02.04.2004 FR 0450670
(43) Date de publication de la demande: 05.10.2005
(73) Titulaire: L'Oréal, 75008 Paris (FR)
(72) Inventeur: BEBOT, Cécile, 92110, CLICHY (FR); LAURENT, Ludivine, 92400, COURBEVOIE (FR)
(74) Mandataire: Catherine, Alain

(56) Documents cités:
- WO-A-00/44337
- WO-A-99/17719
- GB-A- 880 926
- US-A- 2 643 375
- US-A- 5 957 140
- US-A- 5 968 286

## Description

L'invention concerne un procédé de traitement des fibres capillaires, et l'utilisation dudit procédé pour améliorer la brillance des fibres capillaires.

De nombreux procédés de traitement des cheveux utilisent des composés siliconés. Il est en effet connu que les composés siliconés sont des actifs cosmétiques qui améliorent les propriétés cosmétiques des cheveux. Ils ont un effet conditionneur sur les cheveux et apportent de la brillance.

Cependant, cet apport de brillance apporté par les silicones a tendance à s'estomper rapidement avec le temps.

Par ailleurs, de nombreux procédés de traitement des cheveux utilisent une étape de chauffage des cheveux. Cependant, le chauffage des cheveux peut avoir un effet dégradant sur les fibres.

La demande de brevet WO 99/17719 divulgue un procédé de traitement des cheveux, pour le conditionnement et la mise en forme non permanente des cheveux. Ce procédé comprend l'application d'une composition non rincée comprenant un agent conditionnant siliconé non volatil, une résine et un vecteur, puis l'utilisation d'un appareil chauffant pour sécher ou coiffer les cheveux, cette opération induisant une réduction du module de flexion d'au moins 1%. L'agent conditionnant siliconé non volatil représente de 0,1 à 2% en poids du poids total de la composition.

Le but de la présente invention est donc de fournir un procédé de traitement des fibres capillaires qui remédie aux inconvénients de l'art antérieur.

En particulier, la présente invention a pour but de fournir un procédé de traitement des fibres capillaires qui permet de conférer aux fibres un bon niveau de brillance et qui permet de prolonger cet effet dans le temps.

Ledit procédé doit en outre permettre d'augmenter la raideur et améliorer le lissage des fibres capillaires.

La Demanderesse a trouvé qu'il était possible de remédier aux inconvénients de l'art antérieur et de remplir les objectifs précités en mettant en oeuvre un procédé de traitement des fibres capillaires comprenant une étape d'application sur les fibres capillaires d'une composition comprenant au moins 5% en poids, par rapport au poids total de la composition, d'au moins une silicone choisie parmi les silicones arylées et les gommes de silicone, puis une étape d'élévation de la température des fibres capillaires, au moyen d'un fer plat chauffant, à une température comprise entre 150 et 250°C.

Ainsi, l'invention a pour objet un procédé de traitement des fibres capillaires comprenant les étapes suivantes :
- application sur les fibres capillaires d'une composition comprenant au moins 5% en poids, par rapport au poids total de la composition, d'au moins une silicone choisie parmi les silicones arylées et les gommes de silicone, puis
- élévation de la température des fibres capillaires, au moyen d'un fer plat chauffant, à une température comprise entre 150 et 250°C.

De préférence, ladite composition est appliquée sur des fibres capillaires humides.

Avantageusement, les fibres sont partiellement préséchées.

La ou les silicones présentes dans la composition sont choisies parmi les silicones arylées et les gommes de silicone.

Par silicone arylée, on entend une silicone dont au moins un des motifs de répétition comprend au moins un groupe aryle.

Parmi les silicones arylées utilisables dans le procédé selon l'invention, on peut citer les polyalkylarylsiloxanes et les polydiarylsiloxanes.

Parmi les polyalkylarylsiloxanes, on peut citer en particulier les polyphényltriméthylsiloxanes (encore appelés phényltriméthicones) de formule générale : 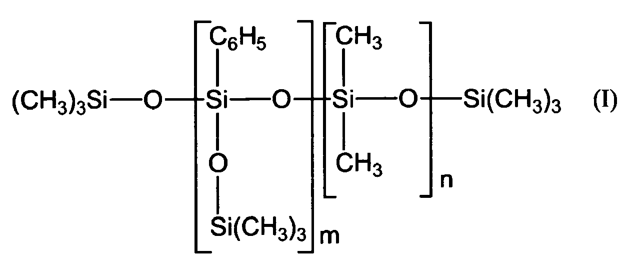 dans laquelle :
n est un nombre entier allant de 0 à 100, et
m est un nombre entier allant de 1 à 400.

Une des variantes consiste à utiliser des composés de formule (I) pour lesquels n est égal à zéro et m est un nombre entier allant de 1 à 3.

Parmi les polyphényltriméthylsiloxanes de formule (I) pour lesquels n est égal à zéro utilisables selon l'invention, on peut citer par exemple le produit commercialisé par la société DOW CORNING sous la référence DC 556 FLUID.

Parmi les polyphényltriméthylsiloxanes de formule (I) pour lesquels n est différent de zéro, utilisables selon l'invention, on peut citer par exemple les produits commercialisés par la société GOLDSCHMIDT sous les références ABIL AV 200 et ABIL AV 1000.

Les gommes de silicone utilisables dans le procédé selon l'invention peuvent être choisies parmi :
- les polydiméthylsiloxanes (encore appelés diméthicones) de haut poids moléculaire de formule générale : 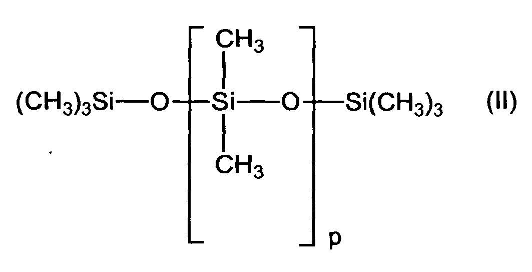
dans laquelle p est un nombre entier supérieur à 2000 ; on peut citer en particulier le produit commercialisé par la société GENERAL ELECTRIC sous la référence VISCASIL 60M ; et
- les polydiméthylsiloxane-αω-silanols (encore appelés diméthiconol) de formule générale : 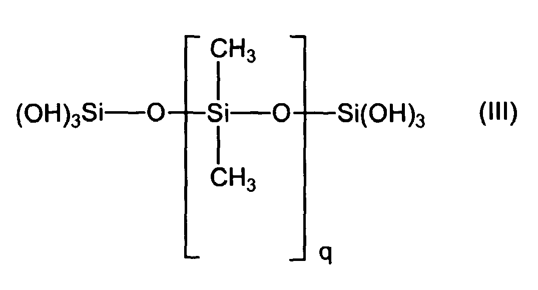
dans laquelle q est un nombre entier supérieur à 2000 ; on peut citer en particulier les produits commercialisés par la société DOW CORNING sous les références DC SGM-3, Q2-1403, DC F2-1671 et DC 2-9071.

La composition utilisée dans le procédé selon l'invention comprend généralement un ou plusieurs solvants cosmétiquement acceptables, choisis de préférence parmi l'eau, les alcools en C₁-C₆, de préférence les alcanols tels que l'éthanol, le propanol et l'isopropanol, les alcanediols tels que le propylène glycol, le glycérol et le pentanediol, l'alcool benzylique, les éthers de polyols, les esters en C₂-C₆, la N-méthylpyrrolidone (NMP), les cétones en C₃-C₆, et les huiles de silicone.

Les huiles de silicone utilisables comme solvant peuvent être choisies par exemple parmi les cyclométhicones de formule générale : 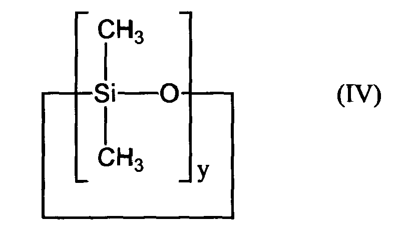 où y est un entier compris entre 3 et 8.

Parmi les cyclométhicones particulièrement préférées, on peut citer le cyclotétradiméthylsiloxane (y = 4), le cyclopentadiméthylsiloxane (n = 5) et le cyclohexadiméthylsiloxane (n = 6).

On peut notamment utiliser les produits vendus par la société DOW CORNING sous les dénominations DC FLUID 244, DC FLUID 245, DC FLUID 344 et DC FLUID 345.

De préférence, la composition appliquée selon le procédé selon l'invention est anhydre.

De préférence encore, le solvant utilisé est l'éthanol ou une huile de silicone.

Dans le but d'améliorer les propriétés cosmétiques des fibres capillaires ou encore d'atténuer ou d'éviter leur dégradation, la composition utilisée selon l'invention peut également comprendre un ou plusieurs actifs cosmétiques supplémentaires.

Ce ou ces actifs sont généralement choisis parmi les silicones organomodifiées telles que les silicones aminées, les polymères cationiques, anioniques, amphotères ou non ioniques, les peptides et leurs dérivés, les hydrolysats de protéines, les cires, les agents de gonflement et de pénétration tels que le diméthylisosorbitol, l'urée et ses dérivés, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les colorants, les filtres solaires, les vitamines ou provitamines, les acides gras, les alcools gras, les huiles minérales, végétales ou synthétiques, ainsi que les parfums et les conservateurs, et leurs mélanges.

Généralement, le ou lesdits actifs cosmétiques supplémentaires représentent de 0,01 à 30%, de préférence de 0,1 à 10%, en poids du poids total de la composition cosmétique.

Le pH de la composition est généralement compris entre 2 et 13, de préférence entre 4 et 10.

Le réglage du pH de la composition peut être obtenu à l'aide d'un agent alcalin, tel que, par exemple, l'ammoniaque, la monoéthanolamine, la diéthanolamine, la triéthanolamine, la 1,3-propanediamine, un carbonate ou bicarbonate alcalin ou d'ammonium, un carbonate organique tel que le carbonate de guanidine, un hydroxyde alcalin, ou bien à l'aide d'un agent acidifiant tel que par exemple l'acide chlorhydrique, l'acide acétique, l'acide lactique, l'acide oxalique ou l'acide borique.

La composition utilisée dans le procédé selon l'invention peut se présenter sous la forme d'une lotion, épaissie ou non, d'une crème ou d'un gel ou d'une mousse.

De préférence, la composition n'est pas conditionnée sous la forme d'un aérosol.

Généralement, la composition appliquée sur les fibres capillaires est appliquée à hauteur de 0,05 à 0,3 g, de préférence de 0,1 à 0,2 g de composition par gramme de fibre capillaire sèche.

Après application de la composition, et avant l'élévation de la température des fibres capillaires au moyen d'un fer plat chauffant, on peut laisser poser ladite composition, généralement pendant 30 secondes à 60 minutes, de préférence 5 à 45 minutes.

Comme expliqué précédemment, le procédé selon l'invention comprend, après l'étape d'application de la composition, une étape d'élévation de la température des fibres capillaires, au moyen d'un fer plat chauffant, à une température comprise entre 150°C et 250°C.

Au sens de la présente invention, on entend par fer un dispositif de chauffage des fibres capillaires mettant en contact lesdites fibres et le dispositif de chauffage.

L'extrémité du fer venant en contact avec les cheveux présente deux surfaces planes. Ces deux surfaces planes peuvent être métalliques. Elles peuvent être lisses ou crantées.

A titre d'exemple de fers utilisables dans le procédé selon l'invention, on peut citer tous types de fer plats et, en particulier, de manière non limitative, ceux décrits dans les brevets US 5 957 140, et US 5 046 516.

L'application du fer peut se faire par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage partiel des fibres capillaires avant l'étape d'élévation de la température, de manière à éviter d'importants dégagements de vapeurs qui pourraient brûler les mains du coiffeur et le cuir chevelu du sujet. Cette étape de pré-séchage peut se faire par exemple au moyen d'un séchoir, d'un casque, ou bien encore par séchage libre.

Comme expliqué précédemment, le procédé selon l'invention permet d'améliorer le niveau de brillance des cheveux et de prolonger cet effet dans le temps.

La présente invention a donc également pour objet l'utilisation du procédé tel que décrit précédemment pour augmenter le niveau de brillance des fibres capillaires et prolonger la brillance dans le temps.

L'invention est illustrée par les exemples suivants.

### Exemple :

On met en oeuvre le procédé de traitement des fibres capillaires selon l'invention en utilisant une composition comprenant un composé siliconé.

Les compositions testées sont les suivantes.

### Composition 1

| | |
|---|---|
| DC 2-9071 | 7% MA |
| Ethanol | 5% |
| Parfum et conservateurs | 0.8% |
| DC 245 FLUID | QS 100 |

| | |
|---|---|
| M.A : Matière Active | |

### Composition 2

| | |
|---|---|
| DC 556 FLUID | 7% |
| Parfum | 0.4% |
| Ethanol | QS 100 |

Les cheveux sont traités selon le procédé suivant :
- application de la composition sur les cheveux humides, la composition représentant environ 0,15 g par gramme de cheveux secs ;
- pré-séchage des cheveux au sèche-cheveux jusqu'à ce que les cheveux soient presque secs (on parle de pré-séchage à 80%) ;
- passage d'un fer plat (fer commercialisé sous la référence Techniliss Ioni Ceramic # PNC 228 par la société VELECTA), en un mouvement continu de la racine des cheveux à la pointe, en un ou plusieurs passages ; la température du fer est d'environ 210°C.

On obtient en final une belle brillance durable sur les cheveux.

## Revendications

1. Procédé de traitement des fibres capillaires **caractérisé en ce qu'**il comprend les étapes suivantes :
- application sur les fibres capillaires d'une composition comprenant au moins 5% en poids, par rapport au poids total de la composition, d'au moins une silicone choisie parmi les silicones arylées et les gommes de silicone, puis
- élévation de la température des fibres capillaires, au moyen d'un fer plat chauffant, à une température comprise entre 150 et 250°C.

2. Procédé selon la revendication 1 **caractérisé en ce que** la composition est appliquée sur des fibres capillaires humides.

3. Procédé selon l'une quelconque des revendications précédentes **caractérisé en ce que** les silicones arylées sont choisies parmi les polyalkylarylsiloxanes et les polydiarylsiloxanes.

4. Procédé selon la revendication 3 **caractérisé en ce que** les polyalkylarylsiloxanes sont choisis parmi les polyphényltriméthylsiloxanes de formule générale : 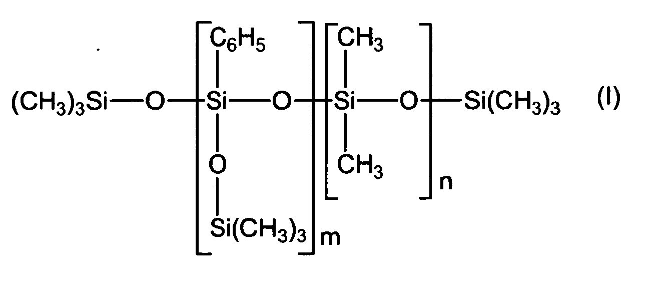 dans laquelle :
n est un nombre entier allant de 0 à 100, et
m est un nombre entier allant de 1 à 400.

5. Procédé selon la revendication 4 **caractérisé en ce que** dans la formule (I) n est égal à zéro et m est un nombre entier allant de 1 à 3.

6. Procédé selon l'une quelconque des revendications précédentes **caractérisée en ce que** les gommes de silicone sont choisies parmi :
- les polydiméthylsiloxanes de formule générale 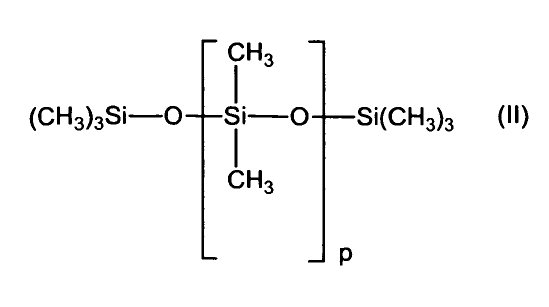 dans laquelle p est un nombre entier supérieur à 2000 ; et
- les polydiméthylsiloxane- αω-silanols de formule générale : 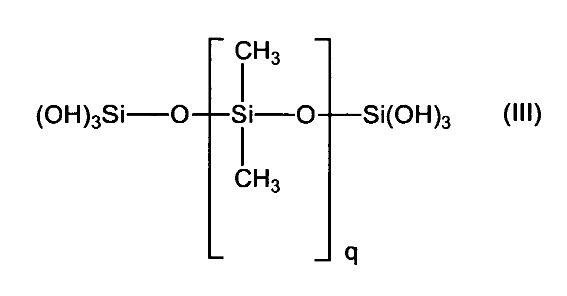
dans laquelle q est un nombre entier supérieur à 2000.

7. Procédé selon l'une quelconque des revendications précédentes **caractérisé en ce que** la composition comprend un ou plusieurs solvants cosmétiquement acceptables choisis parmi l'eau, les alcools en C₁-C₆, de préférence les alcanols tels que l'éthanol, le propanol et l'isopropanol, les alcanediols tels que le propylène glycol, le glycérol et le pentanediol, l'alcool benzylique, les éthers de polyols, les esters en C₂-C₆, la N-méthylpyrrolidone (NMP), les cétones en C₃-C₆, et les huiles de silicone.

8. Procédé selon l'une quelconque des revendications précédentes **caractérisé en ce que** la composition est anhydre.

9. Procédé selon l'une quelconque des revendications précédentes **caractérisé en ce que** la composition comprend en outre un ou plusieurs actifs cosmétiques supplémentaires choisis parmi les silicones organomodifiés, les polymères cationiques, non ioniques, anioniques ou amphotères, les peptides et leurs dérivés, les hydrolysats de protéines, les cires, les agents de gonflement et de pénétration, les agents tensioactifs anioniques, cationiques, non ioniques, amphotères ou zwittérioniques, les agents antichute, les agents antipelliculaires, les épaississants naturels ou synthétiques, associatifs ou non, les agents de suspension, les agents séquestrants, les agents opacifiants, les colorants, les filtres solaires, les vitamines ou provitamines, les acides gras, les alcools gras, les huiles minérales, végétales ou synthétiques, ainsi que les parfums et les conservateurs, et leurs mélanges.

10. Procédé selon la revendication 9 **caractérisé en ce que** le ou lesdits actifs cosmétiques supplémentaires représentent de 0,01 à 30%, de préférence de 0,1 à 10%, en poids du poids total de la composition cosmétique.

11. Procédé selon l'une quelconque des revendications précédentes **caractérisé en ce que** le pH de la composition est compris entre 2 et 13, de préférence entre 4 et 10.

12. Procédé selon l'une quelconque des revendications précédentes **caractérisé en ce que** la composition se présente sous la forme d'une lotion, épaissie ou non, d'une crème ou d'un gel ou d'une mousse, à l'exclusion d'un conditionnement sous la forme d'un aérosol.

13. Procédé selon l'une quelconque des revendications précédentes **caractérisé en ce que**, après l'application de la composition et avant l'élévation de la température des fibres capillaires au moyen d'un fer plat chauffant, on laisse poser ladite composition pendant un temps compris entre 30 secondes et 60 minutes.

14. Procédé selon l'une quelconque des revendications précédentes **caractérisé en ce qu'**il comprend entre l'étape d'application sur les fibres capillaires de la composition et l'étape d'élévation de la température des fibres capillaires, une étape de pré-séchage partiel des fibres capillaires.

15. Utilisation du procédé défini selon l'une quelconque des revendications précédentes pour augmenter le niveau de brillance des fibres capillaires et prolonger la brillance dans le temps.

## Claims

1. A hair fiber treating method, **characterized in that** it comprises the following steps:
- an application step onto the hair fibers of a composition comprising at least 5% by weight, as compared to the total weight of the composition, of at least one silicone selected from aryl silicones and silicone gums, then
- a hair fiber temperature rising step, by means of a heating flat iron, at a temperature ranging from 150 to 250°C.

2. A method according to claim 1, **characterized in that** the composition is applied onto damp hair fibers.

3. A method according to anyone of preceding claims, **characterized in that** aryl silicones are selected from polyalkylarylsiloxanes and polydiarylsiloxanes.

4. A method according to claim 3, **characterized in that** polyalkylarylsiloxanes are selected from polyphenyltrimethylsiloxanes having following general formula: 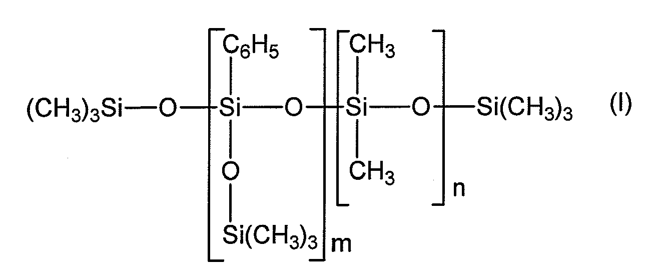 wherein:
n is an integer from 0 to 100, and
m is an integer from 1 to 400.

5. A method according to claim 4, **characterized in that** in formula (I) n is zero and m is an integer from 1 to 3.

6. A method according to anyone of preceding claims, **characterized in that** silicone gums are selected from:
- polydimethylsiloxanes having following general formula: 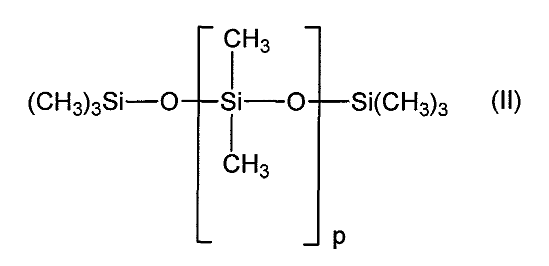 wherein p is an integer greater than 2000;
and
- polydimethylsiloxane- αω-silanols having following general formula: 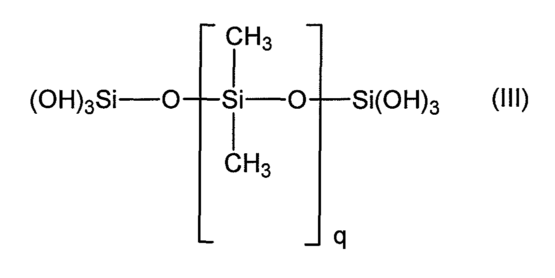
wherein q is an integer greater than 2000.

7. A method according to anyone of preceding claims, **characterized in that** the composition comprises one or more cosmetically acceptable solvent(s) selected from the group consisting of water, C₁-C₆ alcohols, preferably alkanols such as ethanol, propanol and isopropanol, alkanediols such as propyleneglycol, glycerol and pentanediol, benzyl alcohol, polyol ethers, C₂-C₆ esters, N-methylpyrrolidone (NMP), C₃-C₆ cetones and silicone oils.

8. A method according to anyone of preceding claims, **characterized in that** the composition is anhydrous.

9. A method according to anyone of preceding claims, **characterized in that** the composition also comprises one or more additional cosmetic active agent(s) selected from the group consisting of organomodified silicones, cationic, anionic, amphoteric or non ionic polymers, peptides and derivatives thereof, protein hydrolyzates, waxes, swelling agents and penetrating agents, anionic, cationic, non ionic, amphoteric or zwitterionic surfactants, active agents combating hair loss, anti-dandruff agents, natural or synthetic, associative or unassociative thickeners, suspension agents, sequestering agents, opacifying agents, dyes, sunscreen agents, vitamins and provitamins, fatty acids, fatty alcohols, mineral, vegetal or synthetic oils, as well as fragrances and preserving agents, and any combination thereof.

10. A method according to claim 9, **characterized in that** said one or more additional cosmetic active agent(s) represent(s) from 0.01 to 30%, preferably from 0.1 to 10%, by weight, as compared to the total weight of the cosmetic composition.

11. A method according to anyone of preceding claims, **characterized in that** composition pH is between 2 and 13, preferably between 4 and 10.

12. A method according to anyone of preceding claims, **characterized in that** the composition is in the form of a lotion thickened or not, a cream, a gel or a foam, but not in the form of an aerosol.

13. A method according to anyone of preceding claims, **characterized in that**, after applying the composition and before the hair fiber temperature rising step by means of a heating flat iron, said composition is left on, for a period of time ranging from 30 seconds to 60 minutes.

14. A method according to anyone of preceding claims, **characterized in that** it comprises between the application step onto the hair fibers of the composition and the hair fiber temperature rising step, a hair fiber partial pre-drying step.

15. Use of the method according to anyone of preceding claims, to improve the shine intensity of hair fibers while prolonging this effect over time.

## Patentansprüche

1. Verfahren zur Behandlung der Haare, **dadurch gekennzeichnet, dass** es die folgenden Stufen umfasst:
- Auftragen einer Zusammensetzung, die wenigstens 5 Gew.-%, bezogen auf das Gesamtgewicht der Zusammensetzung, wenigstens eines Silicons, ausgewählt aus arylierten Siliconen und Silicongummi, auf die Haare, dann
- Erhöhung der Temperatur der Haare mit Hilfe eines Glätteisens auf eine Temperatur zwischen 150 und 250°C.

2. Verfahren nach Anspruch 1, **dadurch gekennzeichnet, dass** die Zusammensetzung auf feuchte Haare angewendet bzw. aufgetragen wird.

3. Verfahren nach einem der vorangehenden Ansprüche, **dadurch gekennzeichnet, dass** die arylierten Silicone aus Polyalkylarylsiloxanen und Polydiarylsiloxanen ausgewählt werden.

4. Verfahren nach Anspruch 3, **dadurch gekennzeichnet, dass** die Polyalkylarylsiloxane aus Polyphenyltrimethylsiloxanen der allgemeinen Formel: 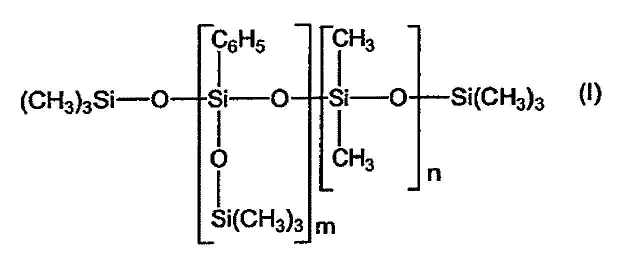 in der:
n eine ganze Zahl von 0 bis 100 ist und
m eine ganze Zahl von 1 bis 400 ist, ausgewählt werden.

5. Verfahren nach Anspruch 4, **dadurch gekennzeichnet, dass** in der allgemeinen Formel (I) n gleich null ist und m eine ganze Zahl von 1 bis 3 ist.

6. Verfahren nach einem der vorangehenden Ansprüche, **dadurch gekennzeichnet, dass** die Silicongummi ausgewählt sind aus:
- Polydimethylsiloxanen der allgemeinen Formel 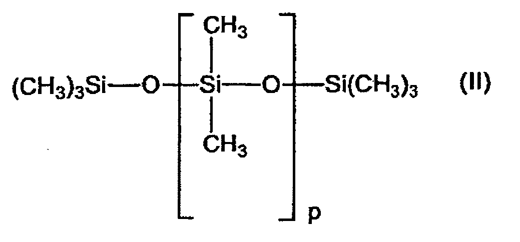 in der p eine ganze Zahl von über 2000 ist, und
- Polydimethylsiloxan-αω-silanolen der allgemeinen Formel: 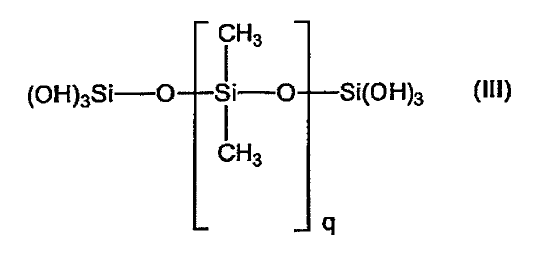 in der q eine ganze Zahl von über 2000 ist.

7. Verfahren nach einem der vorangehenden Ansprüche, **dadurch gekennzeichnet, dass** die Zusammensetzung ein kosmetisch verträgliches Lösungsmittel oder mehrere kosmetisch verträgliche Lösungsmittel umfasst, die ausgewählt sind aus Wasser, C₁-C₆-Alkoholen, vorzugsweise Alkanolen wie Ethanol, Propanol und Isopropanol, Alkandiolen wie Propylenglykol, Glycerin und Pentandiol, Benzylalkohol, Polyolethern, C₂-C₆-Estern, N-Methylpyrrolidon (NMP), C₃-C₆-Ketonen und Silikonölen.

8. Verfahren nach einem der vorangehenden Ansprüche, **dadurch gekennzeichnet, dass** die Zusammensetzung wasserfrei ist.

9. Verfahren nach einem der vorangehenden Ansprüche, **dadurch gekennzeichnet, dass** die Zusammensetzung außerdem einen oder mehrere zusätzliche kosmetische Wirkstoffe umfasst, die ausgewählt sind aus organomodifizierten Silikonen, kationischen, nicht-ionischen, anionischen oder amphoteren Polymeren, Peptiden und ihren Derivaten, Proteinhydrolysaten, Wachsen, Quell- und Penetrationsmitteln, anionischen, kationischen, nicht-ionischen, amphoteren oder zwitterionischen oberflächenaktiven Mitteln, Mitteln gegen Haarausfall, Mitteln gegen Schuppen, natürlichen oder synthetischen assoziativen oder nicht-assoziativen Verdickungsmitteln, Suspensionsmitteln, Sequestriermitteln, Trübungsmitteln, Färbemitteln, Sonnenschutzmitteln, Vitaminen oder Provitaminen, Fettsäuren, Fettalkoholen, mineralischen, pflanzlichen oder synthetischen Ölen sowie Parfüms und Konservierungsmitteln und deren Gemischen.

10. Verfahren nach Anspruch 9, **dadurch gekennzeichnet, dass** der oder die zusätzliche (n) kosmetische(n) Wirkstoff(e) 0,01 bis 30 Gew.-%, vorzugsweise 0,1 bis 10 Gew.-%, bezogen auf das Gesamtgewicht der kosmetischen Zusammensetzung, ausmacht/ausmachen.

11. **Verfahren nach einem der vorangehenden Ansprüche, dadurch gekennzeichnet, dass** der pH der Zusammensetzung zwischen 2 und 13, vorzugsweise zwischen 4 und 10, liegt.

12. Verfahren nach einem der vorangehenden Ansprüche, **dadurch gekennzeichnet, dass** die Zusammensetzung in Form einer Lotion, die verdickt ist oder nicht, einer Creme oder eines Gels oder eines Schaums vorliegt, wobei die Form eines Aerosols ausgeschlossen ist.

13. Verfahren nach einem der vorangehenden Ansprüche, **dadurch gekennzeichnet, dass** man nach Auftragung der Zusammensetzung und vor Erhöhung der Temperatur der Haare mit Hilfe eines Glätteisens die Zusammensetzung während einer Zeit, die zwischen 30 Sekunden und 60 Minuten liegt, ruhen lässt.

14. Verfahren nach einem der vorangehenden Ansprüche, **dadurch gekennzeichnet, dass** es zwischen der Stufe der Auftragung der Zusammensetzung auf die Haare und der Stufe der Temperaturerhöhung der Haare eine Stufe der teilweisen Vortrocknung der Haare umfasst.

15. Verwendung des Verfahrens, das in einem der vorangehenden Ansprüche definiert ist, um den Glanzlevel der Haare zu erhöhen und die Glanzdauer zu verlängern.
